# FASCICULE DE BREVET EUROPEEN

(11) **EP 3 285 702 B1**
(45) Date de publication et mention de la délivrance du brevet: **08.01.2025**
(21) Numéro de dépôt: 16733124.8
(22) Date de dépôt: 08.04.2016
(51) Int. Cl.: A61F 5/02

(54) **DISPOSITIF DE DÉCOMPRESSION VERTÉBRALE**
VORRICHTUNG ZUR VERTEBRALEN DRUCKENTLASTUNG
DEVICE FOR VERTEBRAL DECOMPRESSION

(30) Priorité: 20.04.2015 FR 1553515
(43) Date de publication de la demande: 28.02.2018
(73) Titulaire: Japet Medical Devices, 59120 Loos (FR)
(72) Inventeur: BRATIC, Damien, 75017 Paris (FR); NOEL, Antoine, 59494 Aubry du Hainaut (FR)
(74) Mandataire: Cabinet Beau de Loménie
(86) Numéro de dépôt international: PCT/FR2016/050810
(87) Numéro de publication internationale: WO 2016/170244

(56) Documents cités:
- WO-A2-2005/007046
- US-A- 5 462 518
- US-A- 5 651 764
- US-A1- 2004 077 982
- US-A1- 2009 036 888
- US-A1- 2014 364 784

## Description

### Arrière-plan de l'invention

La présente invention a pour objet un dispositif de décompression vertébrale. Elle trouve en particulier une application pour soulager les personnes souffrant de douleurs dorsales, causées par exemple par une hernie discale. De telles douleurs surviennent, entre autres, du fait que les vertèbres compriment excessivement les disques intervertébraux les séparant les unes des autres.

Un soulagement peut être obtenu en exerçant un effort de distraction sur la colonne vertébrale, de manière à décoapter les vertèbres. Par décoaptation, on entend l'augmentation de l'espace interarticulaire entre deux surfaces trop ajustées l'une à l'autre.

On connaît des dispositifs de décompression vertébrale permettant d'exercer un effort de distraction sur la colonne vertébrale de l'utilisateur.

Un tel dispositif est par exemple proposé par le document EP 0 319 224, qui décrit un dispositif de décompression vertébrale configuré pour entourer une partie du tronc d'un utilisateur, comprenant un support supérieur, un support inférieur et deux barres télescopiques montées sur les supports inférieur et supérieur de manière à exercer une force d'écartement sur les supports inférieur et supérieur. Document US 5 462 518 A décrit un dispositif de décompression vertébrale comprenant des actionneurs disposés en V.

Les barres télescopiques comportent un levier d'actionnement grâce auquel un tiers, tel qu'un professionnel de la santé, peut, lorsque le dispositif est porté par l'utilisateur, éloigner les supports inférieur et supérieur l'un de l'autre, pour exercer un effort de distraction sur la colonne vertébrale de l'utilisateur.

Néanmoins, un tel dispositif est particulièrement encombrant, et ne peut en particulier pas être porté sous des vêtements. En outre, il réduit significativement la mobilité de l'utilisateur, de sorte qu'il ne peut pas être porté plus de quelques minutes. Par ailleurs, il doit être installé et actionné avec l'aide d'un tiers. Il ne permet pas non plus d'adapter la force d'écartement exercée par les barres télescopiques, par exemple en fonction des mouvements réalisés par l'utilisateur, de sorte que, par exemple lorsque l'utilisateur se déplace, l'effort de distraction varie, ce qui tend à limiter le soulagement permis par le dispositif.

### Objet et résumé de l'invention

La présente invention a pour but de résoudre, parmi d'autres, les insuffisances décrites plus haut des dispositifs de décompression vertébrale connus de l'art antérieur, en proposant un dispositif de décompression vertébrale dont l'utilisateur peut s'équiper seul et qu'il peut porter tout au long de la journée, de manière discrète et efficace, l'intensité de l'effort de distraction appliqué sur la colonne vertébrale étant constante, indépendamment de la position ou du déplacement de l'utilisateur.

Ce but est atteint par le fait que l'invention porte sur un dispositif de décompression vertébrale configuré pour entourer le tronc d'un utilisateur, comprenant des moyens de support supérieurs configurés pour entourer une première partie du tronc, des moyens de support inférieurs configurés pour entourer une seconde partie du tronc et des moyens d'élongation montés sur les moyens de support supérieurs et sur les moyens de support inférieurs et configurés pour exercer une force d'écartement sur les moyens de support inférieurs et supérieurs tendant à éloigner les moyens de support inférieurs des moyens de support supérieurs ; en outre, le dispositif comporte un système de commande électronique pour commander les moyens d'élongation en sorte de maintenir un effort de distraction déterminé sur la colonne vertébrale de l'utilisateur.

Considérée dans une position sensiblement verticale de l'utilisateur, la seconde partie du tronc est disposée sous la première partie du tronc.

Par la présence du système de commande électronique, l'utilisateur peut mettre en oeuvre seul le dispositif. En outre, par le maintien d'un effort de distraction déterminé, l'efficacité du dispositif est améliorée, le soulagement dont bénéficie l'utilisateur ne variant pas, en particulier, en fonction de ses déplacements et/ou de sa position.

De préférence, les moyens d'élongation comportent une source d'alimentation, telle qu'une batterie, grâce à laquelle ils peuvent être actionnés sans assistance extérieure.

L'invention est déclinée ci-après dans une série de variantes de réalisation, qui peuvent être considérées seules ou en combinaison avec une ou plusieurs des précédentes.

De préférence, le système de commande électronique comporte un moyen de réglage pour modifier l'intensité de l'effort de distraction.

Par cette disposition, le dispositif, et plus particulièrement l'intensité de l'effort de distraction sur la colonne vertébrale de l'utilisateur, sont adaptés pour soulager des utilisateurs présentant, par exemple, des morphologies différentes ou souffrant de douleurs d'intensités différentes. En outre, la présence du moyen de réglage permet d'adapter le dispositif aux évolutions de la condition de l'utilisateur.

De manière avantageuse, le dispositif de décompression vertébrale comporte en outre des moyens de mesure configurés pour mesurer au moins une caractéristique relative aux mouvements de l'utilisateur.

Avantageusement, le système de commande électronique est configuré pour modifier la force d'écartement exercée par les moyens d'élongation en fonction des mesures réalisées par les moyens de mesure.

Ainsi, la valeur et/ou la direction de la force d'écartement peuvent également être modifiées en fonction des caractéristiques de l'utilisateur mesurées par les moyens de mesure, de manière que l'intensité de l'effort de distraction soit constante, indépendamment, par exemple, des déplacements de l'utilisateur.

De manière avantageuse, le système de commande électronique est configuré pour modifier l'intensité de l'effort de distraction en fonction des mesures réalisées par les moyens de mesure.

On comprend donc que le moyen de réglage est configuré pour modifier l'intensité de l'effort de distraction en fonction de la mobilité de l'utilisateur, ou de sa position.

De préférence, le dispositif de décompression vertébrale comporte en outre des moyens d'enregistrement des mesures réalisées par les moyens de mesure.

Les moyens d'enregistrement permettent ainsi de mémoriser tout ou partie des déplacements et des positions de l'utilisateur pendant la période pendant laquelle il est équipé du dispositif, de manière, par exemple, à identifier les comportements qui peuvent être à l'origine ou contribuer à sa douleur dorsale.

De manière avantageuse, les moyens d'élongation comportent au moins un actionneur linéaire.

Par cette disposition, la compacité, la légèreté et, par suite, le confort d'utilisation du dispositif, ne sont pas compromis. En outre, la précision, tant en termes de valeur que de direction, de la force d'écartement exercée par les moyens d'élongation, et par suite l'efficacité du dispositif pour soulager la douleur ressentie par l'utilisateur, s'en trouvent également améliorées.

Avantageusement, ledit au moins un actionneur linéaire est monté articulé par rapport aux moyens de support supérieurs et/ou aux moyens de support inférieurs.

Par cette disposition, la liberté de mouvement de l'utilisateur n'est pas compromise, lorsqu'il porte le dispositif selon la présente invention. Il peut ainsi aisément se déplacer ou plier, par exemple, la partie supérieure de son corps par rapport à sa partie inférieure, de tels comportements étant significativement limités, voire impossibles, avec les dispositifs connus de l'art antérieur.

De préférence, le système de commande électronique est configuré pour limiter les mouvements relatifs dudit au moins un actionneur linéaire par rapport aux moyens de support supérieurs et/ou aux moyens de support inférieurs.

Par cette disposition, l'utilisateur est empêché de réaliser certains mouvements qui risqueraient d'augmenter la douleur ressentie ou de le blesser.

De manière avantageuse, les moyens d'élongation comportent au moins un premier et un second actionneurs linéaires configurés pour s'étendre de chaque côté du tronc, lorsque le dispositif est porté par l'utilisateur.

La présence de deux actionneurs linéaires de part et d'autre du tronc de l'utilisateur permet de mieux répartir la force d'écartement exercée par les moyens d'élongation sur les moyens de support inférieurs et supérieurs, de manière à augmenter l'efficacité du dispositif, sans compromettre la mobilité de l'utilisateur.

Selon la présente invention les moyens d'élongation comportent deux paires d'actionneurs linéaires configurées pour s'étendre de chaque côté du tronc, lorsque le dispositif est porté par l'utilisateur.

Par cette disposition, la force d'écartement exercée par les moyens d'élongation est encore mieux répartie sur le tronc de l'utilisateur ; en outre, elle permet une meilleure mobilité de l'utilisateur équipé du dispositif selon la présente invention, et une efficacité encore accrue du dispositif.

Les actionneurs linéaires d'une même paire sont inclinés l'un par rapport à l'autre, de manière à encore accroître la liberté de mouvement de l'utilisateur lorsqu'il porte le dispositif de décompression vertébrale selon la présente invention, tout en permettant, par la présence du système de commande électronique, d'empêcher l'utilisateur de réaliser certains mouvements qui risqueraient de lui être douloureux.

Les actionneurs linéaires d'une même paire sont disposés en V.

Avantageusement, la pointe du V formée par les actionneurs linéaires d'une même paire est dirigée vers le bas.

De manière avantageuse, les actionneurs linéaires s'étendent selon une direction inclinée par rapport à l'axe de la colonne vertébrale.

De préférence, les actionneurs linéaires des moyens d'élongation sont configurés pour exercer des forces d'écartement de valeur et direction différentes.

Cette disposition permet de mieux répartir l'effort de distraction sur la colonne vertébrale, de manière à accroître l'efficacité du dispositif selon la présente invention.

Avantageusement, les moyens de support supérieurs et inférieurs sont configurés pour entourer, respectivement, une partie supérieure et une partie inférieure du segment lombaire de la colonne vertébrale de l'utilisateur.

De préférence, les moyens de support supérieurs et inférieurs sont configurés pour être ajustés autour de la portion de tronc qu'ils entourent.

De manière avantageuse, les moyens de support supérieurs et inférieurs comportent une ceinture réglable, configurée pour adapter le dispositif à différentes morphologies d'utilisateur.

Avantageusement, les moyens de support supérieurs et inférieurs présentent une forme sensiblement conique, de manière à augmenter la stabilité de la fixation du dispositif sur le tronc de l'utilisateur, et, par suite, la précision de la localisation et de l'intensité de l'effort de distraction qu'il applique sur la colonne vertébrale de l'utilisateur.

La présente invention porte également sur un système de décompression vertébrale comportant un dispositif de décompression vertébrale selon la présente invention, et un système de traitement de données, le système de commande électronique du dispositif de décompression vertébrale comportant en outre des moyens de transmission sans fil configurés pour transmettre les mesures réalisées par les moyens de mesure au système de traitement.

Par cette disposition, le système est configuré pour permettre un échange de données entre l'utilisateur et un tiers, tel qu'un professionnel de la santé, de manière à ajuster la prescription relative aux conditions d'usage du dispositif, telles que l'intensité de l'effort de distraction.

De préférence, les moyens de transmission sans fil sont configurés pour transmettre au système de traitement les données relatives aux moyens d'élongation, telles que les variations de la valeur et/ou de la direction de la force d'écartement.

De manière avantageuse, les moyens de transmission sans fil sont configurés pour transmettre au système de traitement les données enregistrées par les moyens d'enregistrement.

Par cette disposition, le système de traitement peut traiter des données relatives à une période significative d'utilisation du dispositif selon la présente invention.

Avantageusement, le système de traitement comporte un dispositif de stockage configuré pour enregistrer les données transmises par les moyens de transmission sans fil du système de commande électronique.

Ainsi, le tiers peut comparer, lors d'échanges successifs avec le dispositif, les données reçues, de manière, par exemple, à évaluer l'efficacité des changements de paramètres appliqués.

### Brève description des dessins

D'autres caractéristiques et avantages de l'invention apparaîtront plus clairement et de manière complète à la lecture de la description ci-après d'un mode de réalisation préféré, donné à titre d'exemple non limitatif et en référence aux dessins annexés suivants sur lesquels :
- les figures **1** et **2** représentent schématiquement un exemple de dispositif de décompression vertébrale selon la présente invention, respectivement vu de derrière et de devant ;
- la figure **3** représente schématiquement une vue partiellement éclatée du dispositif selon la présente invention ; et

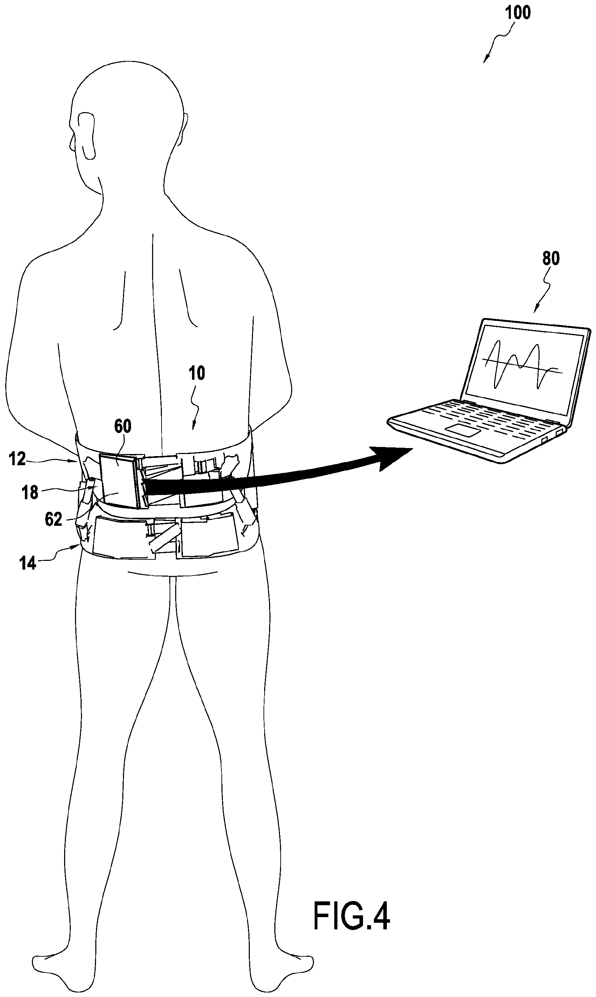

- la figure **4** représente schématiquement un système de décompression vertébrale selon la présente invention.

### Description détaillée de l'invention

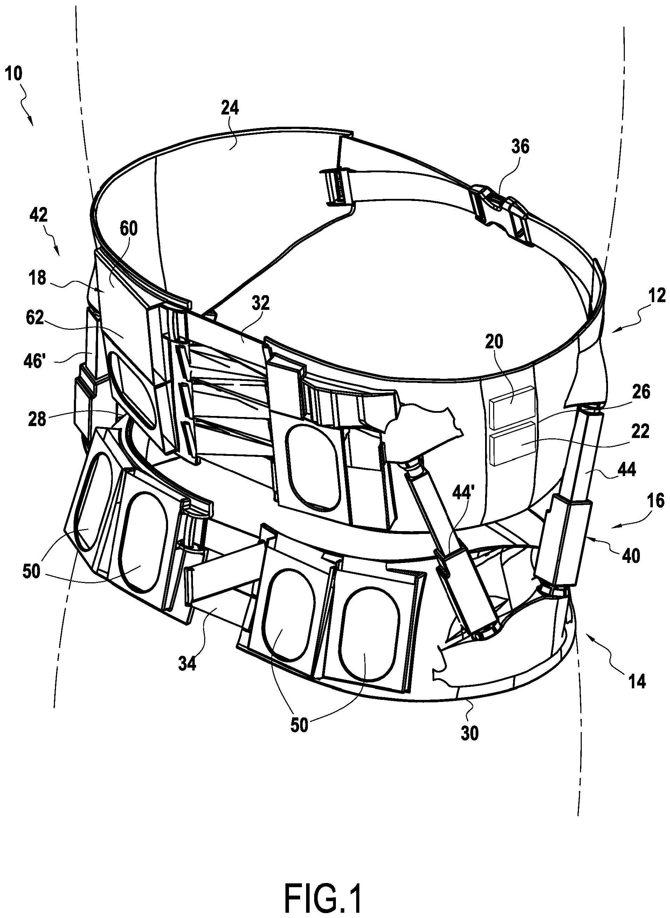

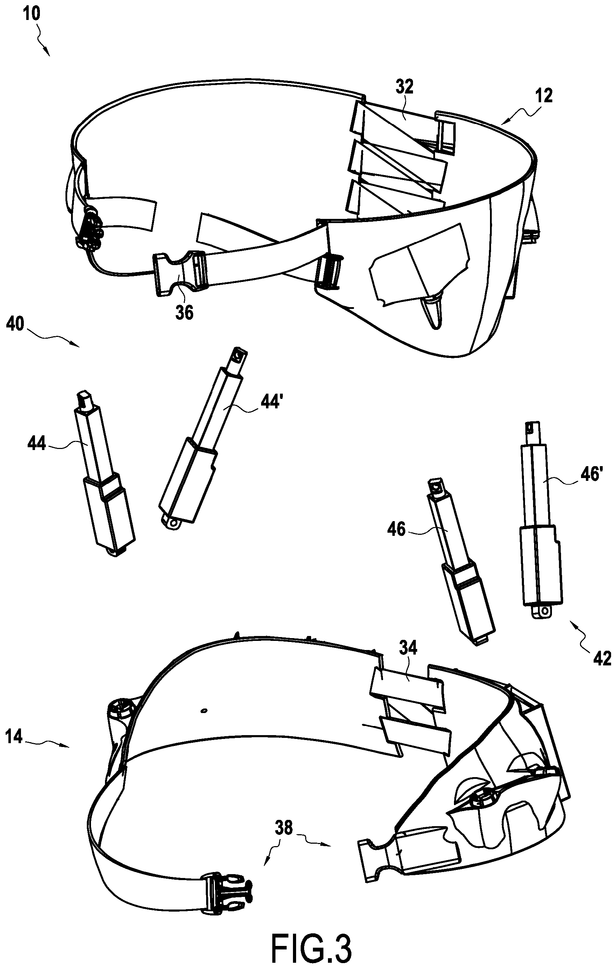

Dans l'exemple représenté aux figures **1 à 3****,** le dispositif de décompression vertébrale **10** selon la présente invention comprend des moyens de support supérieurs **12,** des moyens de support inférieurs **14** et des moyens d'élongation **16** qui, tel que cela sera détaillé dans la suite de la description, sont montés à la fois sur les moyens de support supérieurs **12** et sur les moyens de support inférieurs **14,** les moyens d'élongation **16** étant configurés pour exercer une force d'écartement sur les moyens de support inférieurs et supérieurs **14, 12,** tendant à éloigner les moyens de support inférieurs **14** des moyens de support inférieurs **12.**

Le dispositif de décompression vertébrale **10** comporte en outre un système de commande électronique **18** fixé, par exemple et de manière non limitative, sur les moyens de support supérieurs **12.**

Le dispositif **10** comporte également des moyens de mesure **20** et des moyens d'enregistrement **22** qui seront décrits plus en détail par la suite.

Les moyens de support supérieurs **12** et les moyens de support inférieurs **14** comportent différentes portions de textile **24, 26, 28, 30,** reliées, par exemple, et de manière non limitative, par des bandes élastiques **32, 34,** configurées pour adapter les moyens de support supérieurs et inférieurs **12, 14** à la morphologie de l'utilisateur.

En outre, les moyens de support supérieurs et inférieurs présentent des moyens de fermeture **36, 38,** en l'espèce une sangle clipsable, permettant de faciliter l'équipement de l'utilisateur.

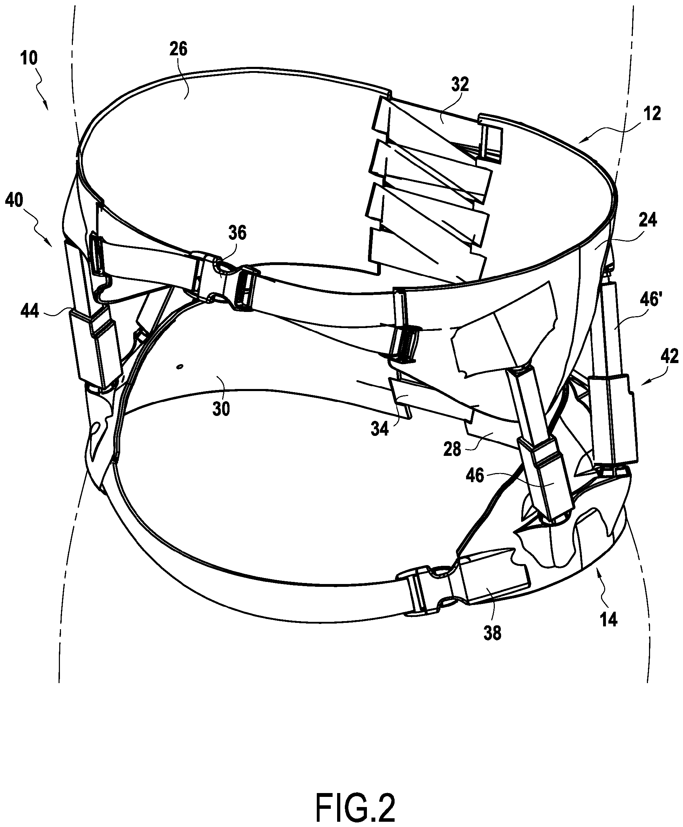

Les figures **1** et **2** représentent en particulier le dispositif **10** porté par un utilisateur, dont la silhouette est symbolisée par des traits mixtes.

Les moyens de support supérieurs **12** présentent ainsi la forme d'une ceinture, configurée pour entourer une première partie du tronc d'un utilisateur ; par exemple et de manière non limitative, les moyens de support supérieurs **12** sont configurés pour entourer la partie médiane du torse de l'utilisateur.

Les moyens de support inférieurs **14** présentent, quant à eux, la forme d'une ceinture, configurée pour entourer une seconde partie du tronc d'un utilisateur, par exemple et de manière non limitative, une partie de la sangle abdominale de l'utilisateur.

On comprend donc que les moyens de support supérieurs **12** et inférieurs **14** sont configurés de manière à entourer une partie de la colonne vertébrale de l'utilisateur, par exemple et de manière non limitative, le segment lombaire de la colonne vertébrale.

Par exemple et de manière non limitative, les moyens de support inférieurs et supérieurs **14, 12** comportent un matériau présentant des propriétés non allergènes ; ils peuvent en outre être revêtus, par exemple, d'une mousse thermoformable, de manière à encore mieux adapter le dispositif de décompression vertébrale **10** à la morphologie de l'utilisateur et à améliorer la coopération entre les moyens de support **12, 14** et le tronc de l'utilisateur.

Par exemple et de manière non limitative, les moyens de support supérieurs **12** et inférieurs **14** présentent une forme conique configurée pour encore améliorer la coopération des moyens de support inférieurs et supérieurs **14, 12** avec le tronc de l'utilisateur.

Tel que représenté sur les différentes figures, les moyens d'élongation **16** comportent une première **40** et une seconde **42** paires d'actionneurs linéaires, les première et seconde paires **40, 42** étant configurées pour s'étendre de chaque côté du tronc, lorsque le dispositif **10** est porté par l'utilisateur.

Par exemple et de manière non limitative, chacune des paires **40, 42** comporte un premier et un second actionneurs linéaires **44, 44', 46, 46'.** Par exemple et de manière non limitative, les différents actionneurs linéaires **44, 44', 46, 46'** sont des vérins électriques ; on pourrait bien évidemment concevoir, et sans sortir du cadre de la présente invention, tout autre type d'actionneur linéaire, tel que par exemple un vérin hydraulique.

Chacun des actionneurs présente une direction longitudinale et une première et une seconde extrémités opposées l'une à l'autre ; tel que représenté en particulier sur les figures **1** et **2****,** l'une des extrémités des actionneurs linéaires est montée sur les moyens de support supérieurs **12,** l'autre extrémité étant montée sur les moyens de support inférieurs **14.**

Par exemple, et de manière non limitative, les actionneurs linéaires **44, 44', 46, 46'** comportent des rotules montées sur chacune de leurs extrémités, et configurées de manière que les différents actionneurs linéaires sont articulés par rapport aux moyens de support supérieurs **12** et aux moyens de support inférieurs **14.**

Tel que représenté sur les différentes figures, le montage des actionneurs linéaires est réalisé par des poches formées sur les moyens de support supérieurs et inférieurs **12, 14,** et plus particulièrement sur leurs portions de textile **24, 26, 28, 30,** les extrémités des différents actionneurs linéaires étant logées dans lesdites poches ; on pourrait bien évidemment concevoir, et sans sortir du cadre de la présente invention, tout autre moyen de montage des actionneurs linéaires sur les moyens de support **12, 14.**

Les moyens d'élongation **16** comportent en outre des batteries **50** montées, par exemple et de manière non limitative, sur la portion arrière des moyens de support supérieurs et inférieurs **12, 14.**

Par portion arrière, on entend la partie du dispositif de décompression vertébrale **10** configurée pour recouvrir une partie du dos de l'utilisateur, lorsque le dispositif **10** est porté.

Les batteries **50** sont configurées de manière à actionner les actionneurs linéaires ; on pourrait concevoir, sans sortir du cadre de la présente invention, toute autre source d'alimentation permettant d'actionner les actionneurs linéaires sans assistance extérieure.

Les actionneurs linéaires sont configurés de manière à exercer une force d'écartement sur les moyens de support supérieurs **12** et inférieurs **14,** l'exercice d'une telle force d'écartement permettant, grâce à l'adhérence des moyens de support supérieurs et inférieurs **12, 14** sur les parties du tronc de l'utilisateur, en éloignant les moyens de support inférieurs **14** des moyens de support supérieurs **12,** d'appliquer un effort de distraction sur la colonne vertébrale de l'utilisateur.

Le système de commande électronique **18** du dispositif **10** est configuré pour commander les moyens d'élongation **16** en sorte de maintenir un effort de distraction déterminé sur la colonne vertébrale de l'utilisateur.

Par exemple et de manière non limitative, le système de commande électronique **18** comporte une plaque électronique permettant de piloter la valeur et/ou la direction de la force d'écartement exercée par les actionneurs linéaires **44, 44', 46, 46'.**

En d'autres termes, le système de commande électronique **18** du dispositif **10** est configuré pour commander les moyens d'élongation **16** de manière à réguler la force d'écartement que les actionneurs linéaires **44, 44', 46, 46'** exercent sur les moyens de support inférieurs et supérieurs **14, 12** ; par régulation, on entend une variation de la valeur de la force d'écartement et/ou une variation de la direction d'application de ladite force d'écartement.

Les moyens de mesure **20** sont configurés pour mesurer au moins une caractéristique relative aux mouvements de l'utilisateur, tels que sa position, son déplacement ou les mouvements relatifs de différentes parties de son corps ; par exemple et de manière non limitative, les moyens de mesure **20** comportent un accéléromètre, un gyromètre, ou tout autre capteur permettant de déterminer la position relative des différentes parties de l'utilisateur.

Le système de commande électronique **18** est par ailleurs configuré pour modifier la valeur et/ou la direction de la force d'écartement exercée par les actionneurs linéaires des moyens d'élongation **16** en fonction des mesures réalisées par les moyens de mesure **20.**

Par exemple, lorsque l'utilisateur effectue un mouvement de marche, l'intensité de l'effort de distraction devant être appliqué sur la colonne vertébrale de l'utilisateur varie ; le système de commande électronique **18** permet ainsi d'ajuster la valeur et/ou la direction de la force d'écartement exercée par les différents actionneurs linéaires **44, 44', 46, 46',** pour tenir compte de la variation de l'intensité de l'effort de distraction.

Les moyens d'enregistrement **22** sont configurés pour enregistrer les mesures réalisées par les moyens de mesure **20.** Un tel enregistrement des mesures trouve en particulier une utilité pour permettre, par exemple, à un professionnel de santé, d'adapter l'intensité de l'effort de distraction devant être appliqué à la colonne vertébrale de l'utilisateur, d'adapter la prescription relative au port du dispositif ou bien encore de dispenser à l'utilisateur des conseils relativement aux mouvements et/ou aux positions qui risqueraient d'accroître la douleur ressentie et/ou de retarder la guérison.

Tel que représenté sur les différentes figures, les premières et secondes paires **40, 42** des actionneurs linéaires présentent chacune une forme en V, la direction longitudinale de chacun des actionneurs linéaires étant inclinée par rapport à l'axe de la colonne vertébrale.

Cette disposition permet, entre autres, de mieux répartir la force exercée par les actionneurs linéaires, sans entraver la liberté de mouvement de l'utilisateur équipé du dispositif **10.**

On comprend donc que le dispositif de décompression vertébrale **10** permet de maintenir un effort de distraction déterminé sur la colonne vertébrale de l'utilisateur, sans pour autant compromettre la mobilité de l'utilisateur. Ainsi, le dispositif de décompression vertébrale **10** selon la présente invention constitue un exosquelette permettant de supporter une partie du dos de l'utilisateur.

Le système de commande électronique **18** du dispositif **10** est en outre configuré pour limiter les mouvements relatifs des actionneurs linéaires **44, 44', 46, 46'** par rapport aux moyens de supports supérieurs et inférieurs **12, 14,** de manière que l'utilisateur ne puisse pas réaliser des mouvements qui risqueraient de le blesser ou d'accroître sa douleur.

Le dispositif **10** selon la présente invention présente un encombrement réduit, de telle sorte qu'il peut être porté en toute discrétion, sous les vêtements habituels de l'utilisateur.

En outre, il offre une liberté de mouvement significative à l'utilisateur et, grâce en particulier aux batteries **50** décrites précédemment, présente une autonomie lui permettant d'être porté pendant plusieurs heures.

De manière à encore accroître la durée d'autonomie du dispositif **10,** on pourrait concevoir, et sans sortir du cadre de la présente invention, des moyens d'élongation **16** comportant des actionneurs semi-actifs, tel que des actionneurs linéaires coopérant avec des ressorts, de tels actionneurs semi-actifs permettant d'emmagasiner tout ou partie de l'énergie libérée par certains mouvements de l'utilisateur et de réutiliser cette énergie emmagasinée lors d'autres mouvements réalisés par l'utilisateur.

Le système de commande électronique **18** comporte par ailleurs des moyens de réglage **60** permettant de modifier l'intensité de l'effort de distraction appliqué sur la colonne vertébrale de l'utilisateur grâce à la force d'écartement des moyens d'élongation **16.** Le moyen de réglage **60** est ainsi configuré pour permettre à un professionnel de la santé d'ajuster le réglage du dispositif **10** à la morphologie et à la douleur ressentie par l'utilisateur.

En outre, le système de commande électronique **18** comporte des moyens de transmission sans fil **62** permettant, entre autres, de transmettre les mesures réalisées par les moyens de mesure **20.**

On peut également concevoir, et sans sortir du cadre de la présente invention, des moyens de transmission sans fil **62** qui sont configurés pour transmettre les variations de valeurs de la force d'écartement exercée par les moyens d'élongation **16** lors de l'utilisation du dispositif **10.**

L'invention porte également sur un système de décompression vertébrale **100** comportant un dispositif de décompression vertébrale **10** selon la présente invention et un système de traitement de données **80,** par exemple installé dans un dispositif informatique tel qu'un ordinateur ou un smartphone, le système de traitement de données **80** étant configuré pour recevoir les données transmises par les moyens de transmission sans fil **62** du système de commande électronique **18.**

Le système de décompression vertébrale permet ainsi à un tiers, tel qu'un professionnel de la santé d'accéder aux données de l'utilisateur sans que celui-ci ne doive nécessairement retirer le dispositif **10** ou même être à proximité du tiers.

Par exemple et de manière non limitative, les moyens de transmission sans fil **62** sont configurés pour transmettre au système de traitement de données **80** les données relatives aux moyens d'élongation **16,** telles que les variations de la valeur et/ou de la direction de la force d'écartement.

Les moyens de transmission sans fil **62** sont également configurés pour transmettre au système de traitement de données **80** les données enregistrées par les moyens d'enregistrement **22 ;** on peut également concevoir la présence d'un dispositif de stockage dans le système de traitement de données **80** configuré pour enregistrer les données transmises par les moyens de transmission sans fil **62** du système de commande électronique **18** du dispositif de décompression vertébrale **10.**

La description ci-dessus est donnée à titre d'exemple, et n'est donc pas limitative de l'invention.

En particulier, l'invention, bien que particulièrement adaptée aux douleurs lombaires, peut également être adaptée pour permettre de soulager tout autre type de douleur dorsale, telles que les douleurs cervicales ou thoraciques.

En outre, le nombre, la structure et la disposition des actionneurs linéaires des moyens d'élongation de la présente invention ne sont aucunement limitatifs de la présente invention.

## Revendications

1. Dispositif de décompression vertébrale (10) configuré pour entourer le tronc d'un utilisateur, comprenant :
- des moyens de support supérieurs (12) configurés pour entourer une première partie du tronc ;
- des moyens de support inférieurs (14) configurés pour entourer une seconde partie du tronc ;
- des moyens d'élongation (16) montés sur les moyens de support supérieurs (12) et sur les moyens de support inférieurs (14) et configurés pour exercer une force d'écartement sur les moyens de support inférieurs et supérieurs tendant à éloigner les moyens de support inférieurs des moyens de support supérieurs ;
le dispositif comportant en outre un système de commande électronique (18) pour commander les moyens d'élongation (16) en sorte de maintenir un effort de distraction déterminé sur la colonne vertébrale de l'utilisateur,
les moyens d'élongation comprenant deux paires d'actionneurs configurées pour s'étendre de chaque côté du tronc, lorsque le dispositif est porté par l'utilisateur,
les les actionneurs d'une même paire étant inclinés l'un par rapport à l'autre et disposés en V, le dispositif étant **caractérisé en ce que** la pointe du V est dirigée vers le bas.

2. Dispositif de décompression vertébrale (10) selon la revendication **1, caractérisé en ce que** le système de commande électronique (18) comporte un moyen de réglage (60) pour modifier l'intensité de l'effort de distraction.

3. Dispositif de décompression vertébrale (10) selon la revendication **1** ou **2, caractérisé en ce qu'**il comporte en outre des moyens de mesure (20) configurés pour mesurer au moins une caractéristique relative aux mouvements de l'utilisateur.

4. Dispositif de décompression vertébrale (10) selon la revendication **3, caractérisé en ce que** le système de commande électronique (18) est configuré pour modifier la force d'écartement exercée par les moyens d'élongation (16) en fonction des mesures réalisées par les moyens de mesure (20).

5. Dispositif de décompression vertébrale (10) selon la revendication **3** ou **4, caractérisé en ce qu'**il comporte en outre des moyens d'enregistrement (22) des mesures réalisées par les moyens de mesure (20).

6. Dispositif de décompression vertébrale (10) selon l'une quelconque des revendications **1 à 5, caractérisé en ce que** les actionneurs des moyens d'élongation (16) sont des actionneurs linéaires (44, 44', 46, 46').

7. Dispositif de décompression vertébrale (10) selon la revendication **6, caractérisé en ce que** lesdits actionneurs linéaires (44, 44', 46, 46') sont montés articulés par rapport aux moyens de support supérieurs (12) et/ou aux moyens de support inférieurs (14).

8. Dispositif de décompression vertébrale (10) selon la revendication **7, caractérisé en ce que** le système de commande électronique (18) est configuré pour limiter les mouvements relatifs desdits actionneurs linéaires (44, 44', 46, 46') par rapport aux moyens de support supérieurs et/ou aux moyens de support inférieurs.

9. Dispositif de décompression vertébrale (10) selon l'une quelconque des revendications **1 à 8, caractérisé en ce que** chaque actionneur s'étend dans une direction qui est inclinée par rapport à l'axe de la colonne vertébrale.

10. Système de décompression vertébrale comportant un dispositif de décompression vertébrale (10) selon l'une quelconque des revendications **1 à 9** et selon la revendication **3,** et un système de traitement de données, le système de commande électronique (18) comportant en outre des moyens de transmission sans fil (62) configurés pour transmettre les mesures réalisées par les moyens de mesure (20) au système de traitement.

## Patentansprüche

1. Wirbelsäulendekompressionsvorrichtung (10), die dazu ausgestaltet ist, den Rumpf eines Benutzers zu umgeben, umfassend:
- obere Stützmittel (12), die dazu ausgestaltet sind, einen ersten Teil des Rumpfes zu umgeben;
- untere Stützmittel (14), die dazu ausgestaltet sind, einen zweiten Teil des Rumpfes zu umgeben;
- Verlängerungsmittel (16), die an den oberen Stützmitteln (12) und an den unteren Stützmitteln (14) montiert und dazu ausgestaltet sind, eine Abstandskraft auf die unteren und oberen Stützmittel auszuüben, die dazu dient, die unteren Stützmittel und die oberen Stützmittel fernzuhalten;
wobei die Vorrichtung ferner ein elektronisches Steuersystem (18) zum Steuern der Verlängerungsmittel (16) beinhaltet, um eine bestimmte Ablenkungsanstrengung auf die Wirbelsäule des Benutzers aufrechtzuerhalten,
wobei die Verlängerungsmittel zwei Paare an Stellgliedern beinhalten, die dazu ausgestaltet sind, sich von jeder Seite des Rumpfes zu erstrecken, wenn die Vorrichtung durch den Benutzer getragen wird,
wobei die Stellglieder eines gleichen Paares in Bezug zueinander geneigt und als V angeordnet sind,
wobei die Vorrichtung **dadurch gekennzeichnet ist, dass** die Spitze des V nach unten gerichtet ist.

2. Wirbelsäulendekompressionsvorrichtung (10) nach Anspruch 1, **dadurch gekennzeichnet, dass** das elektronische Steuersystem (18) ein Einstellmittel (60) beinhaltet, um die Intensität der Ablenkungsanstrengung zu modifizieren.

3. Wirbelsäulendekompressionsvorrichtung (10) nach Anspruch 1 oder 2, **dadurch gekennzeichnet, dass** sie ferner Messmittel (20) beinhaltet, die dazu ausgestaltet sind, zumindest eine Charakteristik relativ zu den Bewegungen des Benutzers zu messen.

4. Wirbelsäulendekompressionsvorrichtung (10) nach Anspruch 3, **dadurch gekennzeichnet, dass** das elektronische Steuersystem (18) dazu ausgestaltet ist, die Abstandskraft, die durch die Verlängerungsmittel (16) ausgeübt wird, in Abhängigkeit von den Messungen, die durch die Messmittel (20) umgesetzt werden, zu modifizieren.

5. Wirbelsäulendekompressionsvorrichtung (10) nach Anspruch 3 oder 4, **dadurch gekennzeichnet, dass** sie ferner Aufzeichnungsmittel (22) für die Messungen, die durch die Messmittel (20) umgesetzt werden, beinhaltet.

6. Wirbelsäulendekompressionsvorrichtung (10) nach einem der Ansprüche 1 bis 5, **dadurch gekennzeichnet, dass** die Stellglieder der Verlängerungsmittel (16) lineare Stellglieder (44, 44', 46, 46') sind.

7. Wirbelsäulendekompressionsvorrichtung (10) nach Anspruch 6, **dadurch gekennzeichnet, dass** die linearen Stellglieder (44, 44`, 46, 46') gelenkig in Bezug auf die oberen Stützmittel (12) und/oder auf die unteren Stützmittel (14) montiert sind.

8. Wirbelsäulendekompressionsvorrichtung (10) nach Anspruch 7, **dadurch gekennzeichnet, dass** das elektronische Steuersystem (18) dazu ausgestaltet ist, die Relativbewegungen der linearen Stellglieder (44, 44', 46, 46') in Bezug auf die oberen Stützmittel und/oder auf die unteren Stützmittel zu begrenzen.

9. Wirbelsäulendekompressionsvorrichtung (10) nach einem der Ansprüche 1 bis 8, **dadurch gekennzeichnet, dass** sich jedes Stellglied in einer Richtung erstreckt, die in Bezug auf die Achse der Wirbelsäule geneigt ist.

10. Wirbelsäulendekompressionssystem, das eine Wirbelsäulendekompressionsvorrichtung (10) nach einem der Ansprüche 1 bis 9 und nach Anspruch 3 und ein Datenverarbeitungssystem beinhaltet, wobei das elektronische Steuersystem (18) ferner Drahtlosübertragungsmittel (62) beinhaltet, die dazu ausgestaltet sind, die Messungen, die durch die Messmittel (20) umgesetzt werden, an das Verarbeitungssystem zu übertragen.

## Claims

1. A vertebral decompression device (10) configured to surround a user's trunk, the device comprising:
- upper support means (12) configured to surround a first portion of the trunk;
- lower support means (14) configured to surround a second portion of the trunk;
- elongation means (16) mounted on the upper support means (12) and the lower support means (14) and configured to exert a separating force on the lower and upper support means tending to move the lower support means away from the upper support means;
the device further including an electronic control system (18) for controlling the elongation means (16) in such a manner as to maintain a determined distraction force on the user's vertebral column, the elongation means comprising two pairs of actuators configured to extend on opposite sides of the trunk when the device is worn by the user, the actuators in a given pair being inclined relative to each other and in a V-arrangement, the device being **characterized in that** the tip of the V-shape points downwards.

2. A vertebral decompression device (10) according to claim 1, **characterized in that** the electronic control system (18) includes regulator means (60) for modifying the magnitude of the distraction force.

3. A vertebral decompression device (10) according to claim 1 or claim 2, **characterized in that** it further includes measurement means (20) configured to measure at least one characteristic relating to the movements of the user.

4. A vertebral decompression device (10) according to claim 3, **characterized in that** the electronic control system (18) is configured to modify the separating force exerted by the elongation means (16) as a function of the measurements taken by the measurement means (20).

5. A vertebral decompression device (10) according to claim 3 or claim 4, **characterized in that** it further includes recording means (22) for recording the measurements taken by the measurement means (20).

6. A vertebral decompression device (10) according to any one of claims 1 to 5, **characterized in that** the actuators of the elongation means (16) are linear actuators (44, 44', 46, 46').

7. A vertebral decompression device (10) according to claim 6, **characterized in that** said linear actuators (44, 44', 46, 46') are hinge-mounted relative to the upper support means (12) and/or to the lower support means (14).

8. A vertebral decompression device (10) according to claim 7, **characterized in that** the electronic control system (18) is configured to limit the movements of said linear actuators (44, 44', 46, 46') relative to the upper support means and/or to the lower support means.

9. A vertebral decompression device (10) according to any one of claims 1 to 8, **characterized in that** each actuator extends in a direction that is inclined relative to the axis of the vertebral column.

10. A vertebral decompression system comprising a vertebral decompression device (10) according to any one of claims 1 to 9 and according to claim 3, together with a data processor system, the electronic control system (18) further comprising wireless transmission means (62) configured to transmit the measurements taken by the measurement means (20) to the processor system.
